# EUROPEAN PATENT APPLICATION

(11) **EP 3 037 053 A1**
(43) Date of publication of application: **29.06.2016**
(21) Application number: 14837188.3
(22) Date of filing: 08.08.2014
(51) Int. Cl.: A61B 18/12, A61B 17/28

(54) **TREATMENT TOOL AND TREATMENT SYSTEM**

(30) Priority: 21.08.2013 US 201361868274 P
(71) Applicant: OLYMPUS CORPORATION, Shibuya-ku Tokyo 151-0072 (JP)
(72) Inventor: ICHIKAWA, Hiroaki, Tokyo 151-0072 (JP); HASHIMOTO, Tatsutoshi, Tokyo 151-0072 (JP); KASAHARA, Hideyuki, Tokyo 151-0072 (JP); SOBAJIMA, Hideo, Tokyo 151-0072 (JP); TAKEI, Yusuke, Tokyo 151-0072 (JP)
(74) Representative: Gunzelmann, Rainer
(86) International application number: PCT/JP2014/071073
(87) International publication number: WO 2015/025745

(57) **Abstract**

A treatment device which applies energy to a biological tissue to treat the biological tissue, includes: a first holding surface disposed on a first jaw; a second holding surface disposed on a second jaw; an energy output portion that is disposed in at least one of the first and second holding surfaces, and outputs the energy to the biological tissue held between the first holding surface and the second holding surface; a handle that is supported by an operator; an operating body that is disposed in the handle and into which an operation of bringing the first and second jaws close to each other is inputtable; and an interlocking mechanism that regulates at least one of a closing time until the first and second jaws reach the closed state from the opened state when one operation is input into the operating body for the handle, and a unit closing amount from the opened state toward the closed state, that interlocks at least one of the first and second jaws with the operating body, and that is interposed between the operating body and the handle.

## Description

### Technical Field

This invention relates to a treatment device and a treatment system to treat a biological tissue by use of energy.

### Background Art

For example, in Jpn. Pat. Appln. KOKAI Publication No. 2009-153620, a treatment device is disclosed which is capable of joining biological tissues by applying energy to the tissue, while gradually increasing a holding pressure of a tissue holding portion by an electric actuator.

For example, in Jpn. Pat. Appln. KOKAI Publication No. 2006-288431, a device is disclosed which is capable of coagulating and incising a biological tissue by ultrasonic energy while the tissue is held at a constant pressure.

For example, in Jpn. Pat. Appln. KOKAI Publication No. 1998-155807, a device is disclosed which is capable of performing cauterization without replacing an instrument while an operating field is clearly kept, even when bleeding occurs or smoke is generated by the cauterization during a treatment, by feeding air or water to a pipeline disposed toward a treatment section.

Furthermore, for example, in Jpn. Pat. Appln. KOKAI Publication No. 1999-164835, it is disclosed that a pressure of a holding portion to hold a tissue is gradually increased.

As disclosed in, for example, Journal 2011 of Japan Surgical Association (Vol. 72, 2011, Extra Edition of schedule/excerpts of the 73rd general meeting (issued on October 20, 2011)), caudal pancreatectomy is performed by using an automatic suturing unit in a certain case. In this case, during pancreatic separation, a region planned to be separated is crushed in five minutes, and then separated.

As disclosed in Journal 2011 of Japan Surgical Association described above, when an operation of holding a very soft biological tissue such as a part of a pancreas is performed, for example, a holding portion of a treatment device is changed from an opened state to a closed state in five minutes for the purpose of preventing crush injury and ischemia of the biological tissue, and thus, it is necessary to very slowly operate the treatment device and hold the biological tissue. It is very difficult to perform this operation by each of the devices disclosed in the abovementioned four documents. That is, when a soft tissue such as the pancreas is treated, it is required to take time in slowly holding and treating the soft tissue in a low-invasive treatment.

### Summary of Invention

An object of this invention is to provide a treatment device which takes time in slowly holding a biological tissue that is very soft and easily causes crush injury and ischemia, for example, a part of a pancreas, to apply a constant pressure to the tissue of a treatment object, and a treatment system having the treatment device.

According to a first aspect of the present invention, there is provide a treatment device which applies energy to a biological tissue to treat the biological tissue, includes: first and second jaws that are closable to and separable from each other; a first holding surface that is disposed on the first jaw; a second holding surface that is disposed on the second jaw to face the first holding surface, and is relatively openable and closable between an opened state where the second holding surface is separated from the first holding surface and a closed state where the second holding surface is close to the first holding surface; an energy output portion that is disposed in at least one of the first and second holding surfaces, and outputs the energy to the biological tissue held between the first holding surface and the second holding surface; a handle that is supported by an operator; an operating body that is disposed in the handle and into which an operation of bringing the first and second jaws close to each other is inputtable; and an interlocking mechanism that regulates at least one of a closing time until the first and second jaws reach the closed state from the opened state when one operation is input into the operating body for the handle, and a unit closing amount from the opened state toward the closed state, that interlocks at least one of the first and second jaws with the operating body, and that is interposed between the operating body and the handle.

### Brief Description of Drawings

FIG. 1A is a schematic view showing a treatment system having a treatment device according to first to twelfth embodiments;
FIG. 1B is a schematic perspective view showing a constant load or constant pressure spring disposed in a handle of the treatment device according to the first embodiment;
FIG. 2A is a schematic vertical cross-sectional view showing an opened treatment section and a shaft of the treatment device of the treatment system according to the first to twelfth embodiments;
FIG. 2B is a schematic vertical cross-sectional view showing a closed treatment section and the shaft of the treatment device of the treatment system according to the first to twelfth embodiments;
FIG. 3A is a schematic side view showing a proximal end of the shaft of the treatment device of the treatment system according to the first embodiment, and a state where an opening/closing lever is distant from the handle to open the treatment section;
FIG. 3B is a schematic side view showing the proximal end of the shaft of the treatment device of the treatment system according to the first embodiment, and a state where the opening/closing lever is brought close to the handle to close the treatment section;
FIG. 4A is a schematic view showing a handle of the treatment device of the treatment system according to the second embodiment;
FIG. 4B is a schematic vertical cross-sectional view showing the handle of the treatment device of the treatment system according to the second embodiment;
FIG. 5A is a schematic view showing an opened treatment section of the treatment device of the treatment system according to a third embodiment;
FIG. 5B is a schematic view showing the closed treatment section of the treatment device of the treatment system according to the third embodiment;
FIG. 6A is a schematic view showing a handle of the treatment device having an opened treatment section of the treatment system according to a modification of the third embodiment;
FIG. 6B is a schematic view showing the handle of the treatment device having the closed treatment section of the treatment system according to the modification of the third embodiment;
FIG. 7 is a schematic view showing a handle of the treatment device of the treatment system according to the fourth embodiment;
(A) to (F) in FIG. 8 are schematic views showing, in order, motions of a speed regulator and an escapement of a cam mechanism of the handle of the treatment device of the treatment system according to the fourth embodiment;
FIG. 9A is a schematic view showing a handle of the treatment device of the treatment system according to the fifth embodiment;
FIG. 9B is a schematic view showing a spiral spring disposed in the handle of the treatment device of the treatment system according to the fifth embodiment;
FIG. 10A is a schematic view showing the treatment device having an opened treatment section of the treatment system according to the sixth embodiment;
FIG. 10B is a schematic view showing the treatment device having the closed treatment section of the treatment system according to the sixth embodiment;
FIG. 11A is a schematic view showing the treatment device having an opened treatment section of the treatment system according to the seventh embodiment;
FIG. 11B is a schematic view showing the treatment device having the closed treatment section of the treatment system according to the seventh embodiment;
FIG. 12A is a schematic view showing the treatment device having an opened treatment section of the treatment system according to the eighth embodiment;
FIG. 12B is a schematic vertical cross-sectional view of the treatment device of the treatment system according to the eighth embodiment which is taken along the arrow line 12B-12B in FIG. 12A;
FIG. 12C is a schematic lateral cross-sectional view of the treatment device of the treatment system according to the eighth embodiment which is taken along the arrow line 12C-12C in FIG. 12A;
FIG. 13A is a schematic view showing the treatment device having an opened treatment section of the treatment system according to the ninth embodiment;
FIG. 13B is a schematic view showing a handle of the treatment device of the treatment system according to the ninth embodiment;
FIG. 14A is a schematic view showing the treatment device having an opened treatment section of the treatment system according to a tenth embodiment;
FIG. 14B is a schematic view showing a handle of the treatment device of the treatment system according to the tenth embodiment;
FIG. 15A is a schematic view showing the treatment device having an opened treatment section of the treatment system according to the eleventh embodiment;
FIG. 15B is a schematic view showing a handle of the treatment device of the treatment system according to the eleventh embodiment in a state observed from a direction shown by an arrow 15B in FIG. 15A;
FIG. 16A is a schematic view showing the treatment device having a closed treatment section of the treatment system according to the eleventh embodiment;
FIG. 16B is a schematic view showing the handle of the treatment device of the treatment system according to the eleventh embodiment in a state observed from a direction shown by an arrow 16B in FIG. 16A;
FIG. 17A is a schematic view showing the treatment device having an opened treatment section of the treatment system according to the twelfth embodiment; and
FIG. 17B is a schematic view showing a handle of the treatment device of the treatment system according to the twelfth embodiment.

### Description of Embodiments

Hereinafter, embodiments of this invention will be described with reference to the drawings.

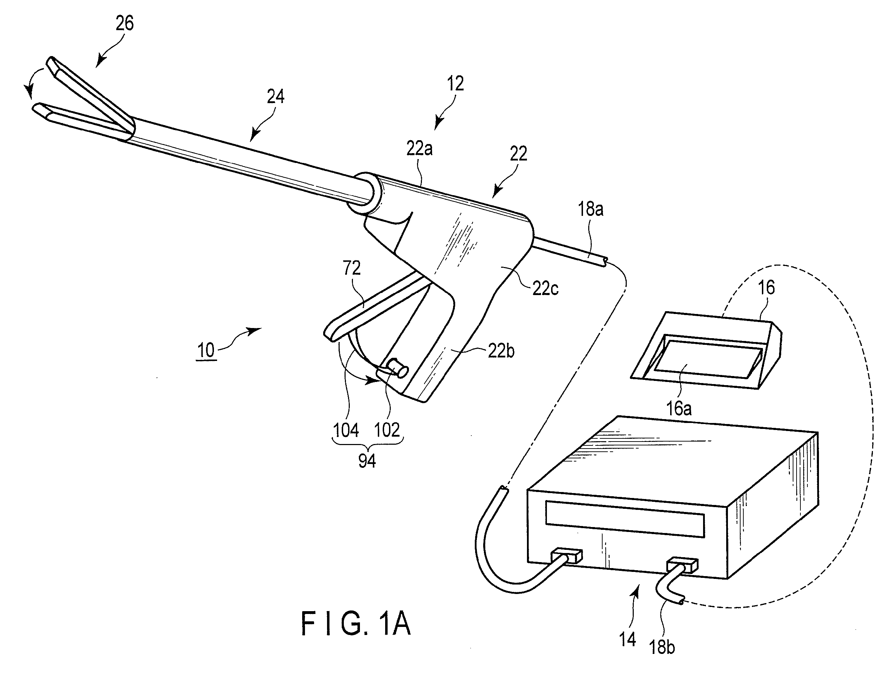

A first embodiment will be described with reference to FIG. 1A to FIG. 3B.

As shown in FIG. 1A, a medical treatment system 10 includes a treatment device 12 and an energy source (a controller) 14. Here, as the treatment device (medical treatment device) 12, there is described, for example, a linear type of surgical treatment device to perform a treatment through an abdominal wall. It is to be noted that the energy source 14 is connected to a foot switch (may be a hand switch) 16 having a pedal 16a by a cable 18b.

The treatment device 12 includes a handle 22 to be supported by an operator, a shaft (an interlocking mechanism) 24 extended from the handle 22 along a central axis C, and an openable/closable treatment section 26. The handle 22 is connected to the energy source 14 via a cable 18a. Consequently, the pedal 16a of the foot switch 16 is operated by the operator, whereby ON/OFF of supply of energy from the energy source 14 to the surgical treatment device 12 is switched. When the pedal 16a is pressed, the energy source 14 outputs the energy on the basis of a state where the energy source 14 is suitably set (a state where an energy output amount, an energy output timing or the like is controlled). When the pressed pedal 16a is released, the output of the energy is forcibly stopped.

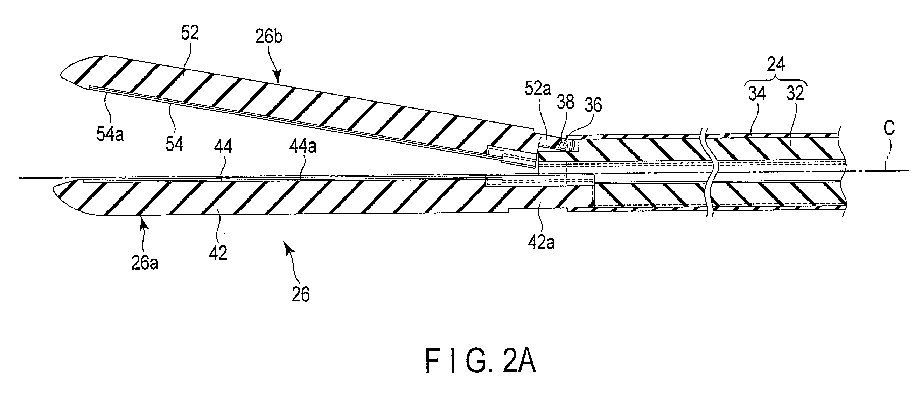

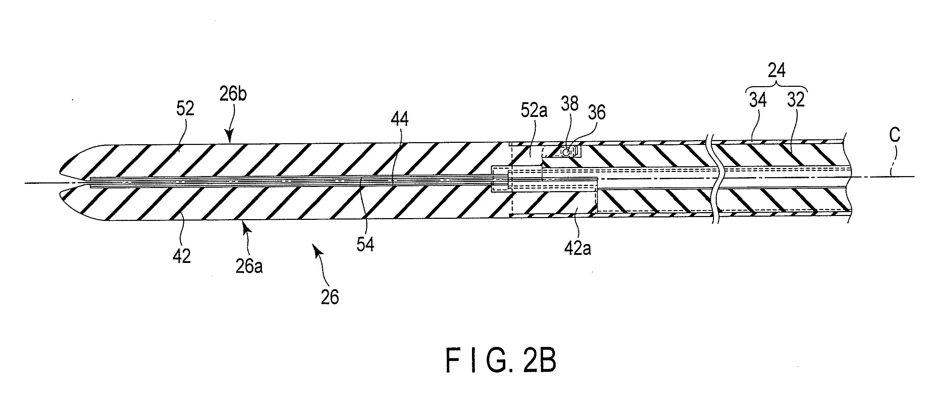

As shown in FIG. 2A and FIG. 2B, the shaft 24 includes an inner cylinder 32 and an outer cylinder 34 slidably disposed on an outer side of the inner cylinder 32. The inner cylinder 32 is fixed to the handle 22 (see FIG. 1A) at a proximal end portion of the inner cylinder 32. The outer cylinder 34 is slidable along an axial direction of the inner cylinder 32.

As shown in FIG. 1A, FIG. 2A and FIG. 2B, the treatment section 26 is disposed at a distal end of the shaft 24. The treatment section 26 includes a first treating portion 26a and a second treating portion 26b. The first treating portion 26a includes a first jaw 42, and a first energy output portion 44 having a first holding surface 44a. The second treating portion 26b includes a second jaw 52, and a second energy output portion 54 having a second holding surface 54a. The first and second jaws 42 and 52 are able to be relatively close to and away from each other. A maximum open width between distal ends of the first and second jaws 42 and 52 is about 30 mm. The first energy output portion 44 is disposed in the first jaw 42, the second energy output portion 54 is disposed in the second jaw 52, and the holding surfaces 44a and 54a of the first and second energy output portions 44 and 54 face each other. The second jaw 52 and the second energy output portion 54 are relatively openable and closable between an opened state where the second jaw 52 and the second energy output portion 54 are away from the first jaw 42 and the first energy output portion 44 and a closed state where the second jaw 52 and the second energy output portion 54 are close to the first jaw 42 and the first energy output portion 44. The first and second jaws 42 and 52 preferably entirely have insulating properties, respectively. As the first and second energy output portions 44 and 54, for example, high-frequency electrodes, heaters or the like are used, respectively, and connected to the energy source 14. Consequently, the biological tissue interposed between the holding surfaces 44a and 54a of the first and second energy output portions 44 and 54 is treated by heat energy.

A proximal portion 42a of the first jaw 42 is fixed to a distal end of the inner cylinder 32 of the shaft 24. On the other hand, a proximal portion 52a of the second jaw 52 is rotatably supported by the distal end of the inner cylinder 32 of the shaft 24 by a support pin (an interlocking mechanism) 36 disposed in a direction orthogonal to an axial direction of the shaft 24. The second jaw 52 rotates in a periaxial direction of the support pin 36, whereby the second jaw 52 is openable and closable to the first jaw 42. Furthermore, the second jaw 52 is urged by an elastic member 38 such as a plate spring, to open from the first jaw 42.

Further, the outer cylinder 34 is slid to the inner cylinder 32, and hence, the proximal portions 42a and 52a of the first jaw 42 and the second jaw 52 can be covered with a distal end of the outer cylinder 34. In this state, as shown in FIG. 2A, the second jaw 52 closes to the first jaw 42 against an urging force of the elastic member 38. On the other hand, when the outer cylinder 34 is slid to a proximal end side of the inner cylinder 32 from the state where the proximal portions 42a and 52a of the first and second jaws 42 and 52 are covered with the distal end of the outer cylinder 34, the second jaw 52 opens from the first jaw 42 by the urging force of the elastic member 38 as shown in FIG. 2B.

The handle 22 is formed into a shape that is easily held by the operator, and is formed into, for example, a substantially L-shape in this embodiment. At one end (a distal end) 22a of the handle 22, a proximal end of the shaft 24 is disposed. For example, the cable 18a described above is extended from the proximal end of the handle 22 that is substantially on the same axis as in the shaft 24.

The other end 22b of the handle 22 is a gripping portion to be gripped by the operator. In the handle 22, a treatment section opening/closing lever (an operating body) 72 is disposed in a handle main body 22c between the one end 22a and the other end 22b so that the lever is disposed in parallel with the other end 22b of the handle. In this embodiment, the treatment section opening/closing lever 72 is disposed on a front side of the other end 22b of the handle 22.

The treatment section opening/closing lever 72 is coupled with a proximal end of the outer cylinder 34 (see FIG. 2A and FIG. 2B) of the shaft 24 in the handle main body 22c that is substantially at a center of the handle 22. When the treatment section opening/closing lever 72 is brought close to and separated from the other end 22b of the handle 22, the outer cylinder 34 moves along an axial direction of the outer cylinder.

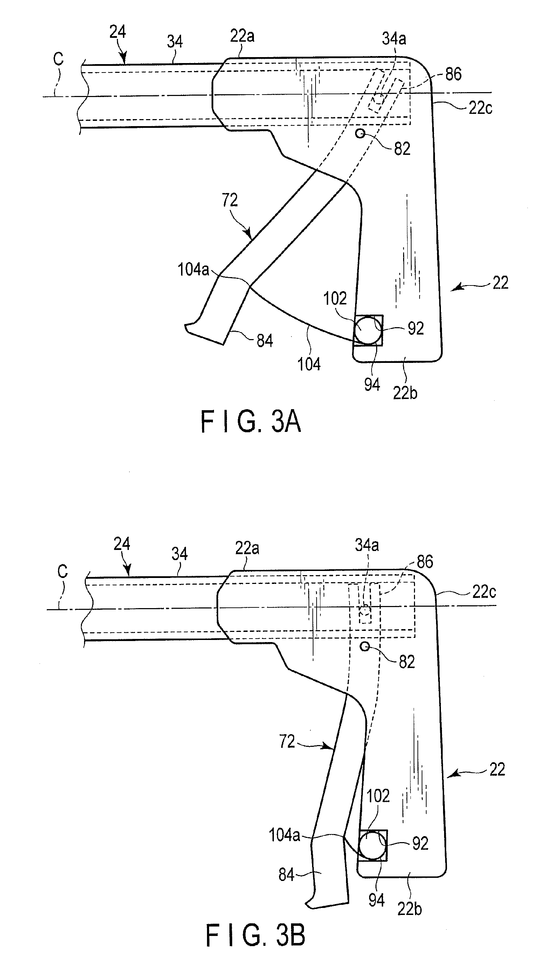

The treatment section opening/closing lever 72 includes a pivot support shaft 82, an operating portion 84, and a pair of claw portions 86. The treatment section opening/closing lever 72 is rotatable in the handle main body 22c by the pivot support shaft 82. The operating portion 84 projects outwardly from a lower end of the handle main body 22c, and is disposed on the front side of the other end 22b of the handle 22. Therefore, the operating portion 84 can be brought close to (see FIG. 3B) and separated from (see FIG. 3A) the other end 22b of the handle 22.

The pair of claw portions 86 are substantially formed into a Y-shape that is present at an upper end of the treatment section opening/closing lever 72 to support the proximal end of the outer cylinder 34, and the claw portions engage with a pin 34a disposed at the proximal end of the outer cylinder 34 of the shaft 24 in the handle main body 22c. The pin 34a extends outwardly in the direction orthogonal to the central axis C to an outer peripheral surface of the outer cylinder 34. Consequently, the pin 34a disposed at the proximal end of the outer cylinder 34 is movably supported to the claw portions 86 at the upper end of the treatment section opening/closing lever 72. In consequence, the outer cylinder 34 is movable to the handle 22 and the inner cylinder 32 by an operation of the operating portion 84 of the treatment section opening/closing lever 72.

The treatment section opening/closing lever 72 is coupled with the after-mentioned proximal end of the outer cylinder 34 of the shaft 24 in a substantially central portion of the handle 22. Consequently, when the treatment section opening/closing lever 72 is brought close to the other end 22b of the handle 22, the after-mentioned outer cylinder 34 of the shaft 24 is advanced to the handle 22 along the axial direction of the outer cylinder 34. On the other hand, when the treatment section opening/closing lever 72 is separated from the other end 22b of the handle 22, the outer cylinder 34 is retreated to the handle 22 along the axial direction of the outer cylinder 34.

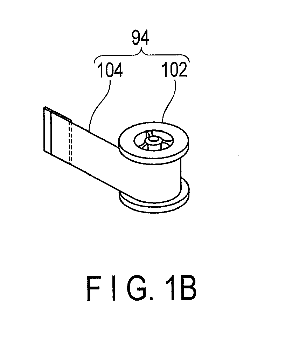

In a front surface of the other end 22b of the handle 22, a receiving portion 92 is formed. An elastic member (an interlocking mechanism) 94 such as a constant-load spring or a constant-pressure spring is disposed in the receiving portion 92. As shown in FIG. 1A and FIG. 1B, as the elastic member 94 according to this embodiment, the constant-load spring or the constant-pressure spring is used, and the elastic member has a drum 102 rotatably supported in the receiving portion 92 of the other end 22b of the handle 22, and a spring material 104 wound around an outer periphery of the drum 102. An extending end portion 104a of the spring material 104 is fixed to the operating portion 84 of the opening/closing lever 72. It is to be noted that a maximum output of the constant-load spring or the constant-pressure spring as the elastic member 94 according to this embodiment can be regulated in accordance with a width or a thickness of the spring material 104. Further, the elastic member 94, in which the constant-load spring or the constant-pressure spring is used, regulates a closing time from when the operating portion 84 of the treatment section opening/closing lever (the operating body) 72 is released in an opened state until a closed state is reached. Here, especially by use of the elastic member (the interlocking mechanism) 94 of the constant-load spring or the constant-pressure spring, a closing amount per unit time is regulated.

Next, an operation of the medical treatment system 10 according to this embodiment will be described.

As shown in FIG. 2B, in the state where the second jaw 52 is closed to the first jaw 42, for example, the treatment section 26 and the shaft 24 of the surgical treatment device 12 are inserted into an abdominal cavity through the abdominal wall. The treatment section 26 of the surgical treatment device 12 is opposed to the biological tissue of the treatment object.

When the opening/closing lever 72 is separated from the other end 22b of the handle 22 in the state where the first and second jaws 42 and 52, i.e., the first and second energy output portions 44 and 54 are closed, the spring material 104 is drawn from the drum 102. In this state, for the purpose of holding the biological tissue between the first and second energy output portions 44 and 54 of the first and second jaws 42 and 52, the handle 22 is operated to operate the treatment section 26.

In this state, the operator releases the opening/closing lever 72. Consequently, by an action of the elastic member 94, e.g., the constant-load spring or the constant-pressure spring, a pulling force works to pull the opening/closing lever 72 toward the other end 22b of the handle 22. Further, the elastic member 94 loads the pulling force on the opening/closing lever 72 at the constant load or the constant pressure. Consequently, depending on regulation of the spring material 104, the closing amount per unit time is regulated, whereby the opening/closing lever 72 of the handle 22 can be moved from the state shown in FIG. 3A to the state shown in FIG. 3B over, for example, a few minutes to about five minutes. Therefore, the outer cylinder 34 interlocking with the action of the opening/closing lever 72 can be advanced as the opening/closing lever 72 of the handle 22 is brought close to the handle 22. Consequently, the second jaw 52 can be closed to the first jaw 42 by use of the support pin (the interlocking mechanism) 36 as a supporting point over a few minutes to about five minutes. Therefore, the biological tissue can be held between the first and second energy output portions 44 and 54 to gradually increase a pressing force over a few minutes to about five minutes. In consequence, for example, when a very soft biological tissue such as a part of the pancreas is held, crush injury or ischemia can be prevented.

In this state, the biological tissue held by operating the pedal 16a of the foot switch 16 while preventing the crush injury or the ischemia can be treated by high-frequency energy or heat energy to be applied to the held biological tissue from the first and second energy output portions 44 and 54.

On the other hand, when the operating portion 84 of the opening/closing lever 72 of the handle 22 is separated from the other end 22b of the handle 22, the jaws can be opened against the urging force of the elastic member 94. That is, by the urging force of the elastic member 38, the second jaw 52 can be opened from the first jaw 42.

As described above, according to this embodiment, the following things can be considered.

According to the treatment device 12 of this embodiment, the elastic member 94 to pull the opening/closing lever 72 toward the other end 22b of the handle 22 is interposed between the opening/closing lever 72 and the other end 22b of the handle 22, and hence, the second jaw 52 can be closed to the first jaw 42 over several minutes to about five minutes by the elastic member 94. That is, when the opening/closing lever 72 is released, the outer cylinder 34 can be prevented from vigorously or rapidly advancing to the inner cylinder 32. Consequently, the first and second jaws 42 and 52 are prevented from being vigorously closed, and take time to be slowly closed. At this time, the operator does not have to continue operating the opening/closing lever 72 of the handle 22 so that the first and second jaws 42 and 52 are prevented from being vigorously closed, and burdens on the operator can noticeably be decreased. The pressure by the pair of holding surfaces to the biological tissue is gradually and slowly increased by taking time, so that a soft biological tissue such as the pancreas can be adjusted to the holding surface to apply a pressure to the tissue. Further, the pressure can be prevented from being locally or noticeably applied to the biological tissue with an edge portion of the holding surface. In addition, the first and second jaws 42 and 52 are relatively closed at the constant load or the constant pressure by the elastic member 94, so that it is possible to inhibit fluctuation of a closing amount per unit time due to operator's power adjustment when the opening/closing lever 72 is brought close to the other end 22b of the handle 22, and unification of quality of a medical treatment can be achieved.

It is to be noted that the elastic member 94 is preferably attachable to and detachable from the receiving portion 92, and is preferably attachable to and detachable from the opening/closing lever 72 of the handle 22. When the elastic member 94 is removed from the receiving portion 92, the first and second jaws 42 and 52 can be opened and closed at free speed by the operation of the opening/closing lever 72.

Additionally, in this embodiment, it has been described that the first energy output portion 44 is disposed in the first jaw 42 and that the second energy output portion 54 is disposed in the second jaw 52, but the two energy output portions 44 and 54 are not necessarily required, and the first energy output portion 44 or the second energy output portion 54 may be disposed. It is to be noted that the first energy output portion 44 and the second energy output portion 54 need to be formed as the holding surfaces to hold the biological tissue.

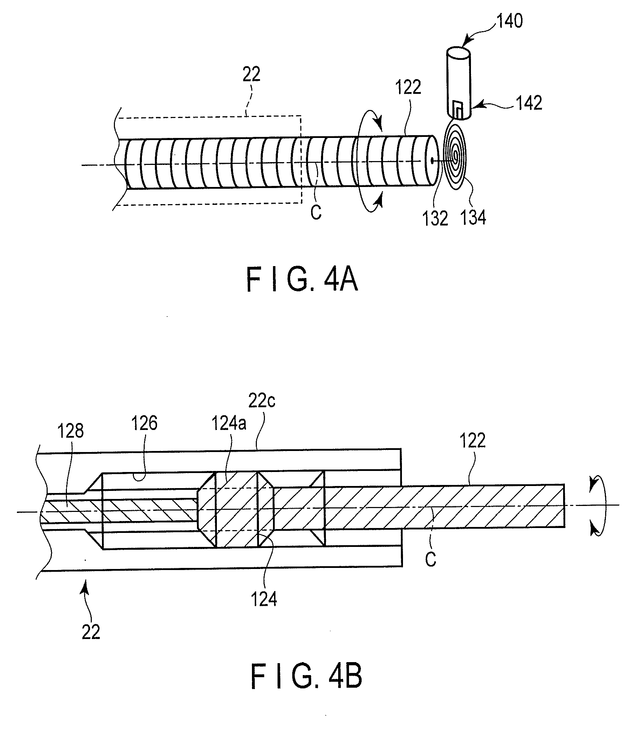

Next, a second embodiment will be described with reference to FIG. 4A and FIG. 4B. This embodiment is a modification of the first embodiment, the same members or members having the same functions as in the first embodiment are denoted with the same reference signs, and detailed descriptions of the members are omitted.

As shown in FIG. 4A and FIG. 4B, a handle 22 of a treatment device 12 according to this embodiment is shaped in the form of a tube. The treatment device 12 according to this embodiment includes a rotary rod (an operating body) 122 in place of the opening/closing lever 72. Into the rotary rod 122, there can be input an operation of bringing first and second jaws 42 and 52 close to each other and separating the jaws from each other. A large diameter portion 124 having an external thread portion 124a is disposed at a distal end the rotary rod 122. The large diameter portion 124 has an outer diameter larger than an outer diameter of the rotary rod 122. It is to be noted that, in a handle 22 (on an inner side), there is formed an internal thread portion 126 into which the external thread portion 124a of the large diameter portion 124 can be screwed.

A movable rod (an interlocking mechanism) 128 as an axially movable body that moves in an axial direction is supported at a further distal end of the large diameter portion 124 of the distal end of the rotary rod 122 in a state where rotation is restricted as the rotary rod 122 moves in the axial direction. The large diameter portion 124 and the movable rod 128 are formed as in a known ball screw structure.

Consequently, when the rotary rod 122 is rotated in a periaxial direction of a central axis C, the movable rod 128 moves in the axial direction of the rod in the same manner as in the first embodiment, and hence, an outer cylinder 34 can relatively be moved to an inner cylinder 32 in an axial direction of the outer cylinder 34.

An elastic member (an interlocking mechanism) 134 such as a spiral spring is disposed via a support shaft 132 at a proximal end of the rotary rod 122. The elastic member 134 includes a locking mechanism 140 that maintains an urged state. The lock mechanism 140 includes a lock pin 142 that is engageable with the elastic member 134. When the lock pin 142 is removed from the lock mechanism 140, the rotary rod 122 rotates in the periaxial direction of the central axis C via the support shaft 132 by the urging force of the elastic member 134. When the rotary rod 122 rotates in the periaxial direction of the central axis C, the outer cylinder 34 advances.

At this time, a rotary motion of the rotary rod 122 is converted into a movement of the axial direction by a ball screw, and hence, the second jaw 52 can close to the first jaw 42 more slowly as compared with a case where the rotary rod 122 is directly moved in the axial direction.

The elastic member (the interlocking mechanism) 134, e.g., the spiral spring according to this embodiment starts to shift from an opened state to a closed state when the lock pin 142 of the lock mechanism 140 is released, and regulates a closing time until the closed state is reached from the opened state. Here, the closing amount per unit time is regulated by especially using the elastic member 134 of the spiral spring.

Consequently, as described in the first embodiment, the first and second jaws 42 and 52 are prevented from being vigorously closed, and take time to be slowly closed. At this time, an operator does not have to continue operating the rotary rod 122 of the handle 22 so that the first and second jaws 42 and 52 are prevented from being vigorously closed, and burdens on the operator can noticeably be decreased. In addition, the first and second jaws 42 and 52 are relatively closed at a substantially constant speed by the elastic member 134, so that it is possible to inhibit fluctuation of the closing amount per unit time due to operator's power adjustment when the rotary rod 122 is rotated to the handle 22 in the periaxial direction of the central axis C, and unification of quality of a medical treatment can be achieved.

It is to be noted that, when the first and second jaws 42 and 52 are opened, the rotary rod 122 may be rotated in an opposite direction to the abovementioned periaxial direction of the central axis C. At this time, an urging force is applied to the elastic member 134 of the spiral spring. Thus, the lock pin 142 of the lock mechanism 140 is engaged with the lock mechanism 140, and hence, the elastic member 134 can be supported in the urged state.

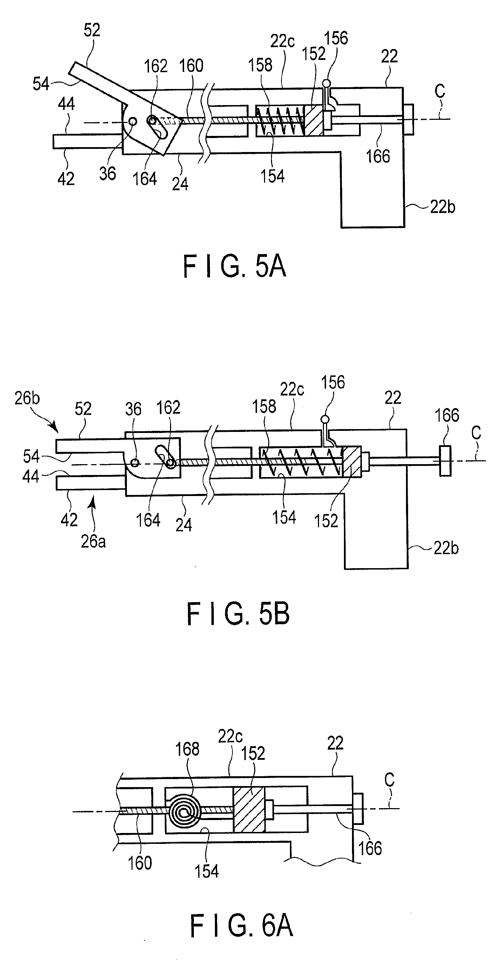

Next, a third embodiment will be described with reference to FIG. 5A and FIG. 5B. This embodiment is a modification of the first and second embodiments, the same members or members having the same functions as in the first and second embodiments are denoted with the same reference signs, and detailed descriptions of the members are omitted.

As shown in FIG. 5A and FIG. 5B, in a treatment device 12 according to this embodiment, for example, a tubular slider receiving portion 154 provided with a slider (an interlocking mechanism) 152 is formed in a handle 22. The slider receiving portion 154 is formed so that the slider 152 can be moved in an axial direction, but moves with difficulty, due to friction between the slider 152 and the slider receiving portion 154, oil sealed in the slider receiving portion 154, or the like. Here, the embodiment is described on the assumption that the friction is present between the slider 152 and the slider receiving portion 154.

A restricting pin (an operating body) 156 that is movable between a restricted position and an opened position to restrict the movement of the slider 152 is supported in the slider receiving portion 154. The restricting pin 156 can be put into and removed from the slider receiving portion 154. Consequently, the restricting pin 156 restricts the slider 152 from moving to a rear end side when the restricting pin is projected into the slider receiving portion 154 as shown in FIG. 5A. On the other hand, the restricting pin 156 allows the slider 152 to move to the rear end side from the restricting pin 156, when the restricting pin is removed from the slider receiving portion 154 as shown in FIG. 5B. However, as to the movement of the slider 152 at this time, the slider is prevented from rapidly moving, by the friction.

Further, a compression coil spring (an interlocking mechanism) 158 is disposed between the slider 152 and a front end of the slider receiving portion 154 in the slider receiving portion 154. A proximal end of a wire (an interlocking mechanism) 160 is supported through a center (e.g., a central axis C) of the compression coil spring 158 on the slider 152. A support pin 162 orthogonal to a longitudinal axis (e.g., the central axis C) of the wire 160 is fixed to a distal end of the wire 160. The support pin 162 is disposed in an engaging hole 164 disposed in a first jaw 42. The compression coil spring 158 urges the slider 152 on the rear end side.

It is to be noted that, in the handle 22, a pressing body 166 is disposed which can move the slider 152 from a position shown in FIG. 5B to a position shown in FIG. 5A, i.e., toward a distal end side against an urging force of the compression coil spring 158. Consequently, when a second jaw 52 is opened from the first jaw 42, the pressing body 166 is moved to a front side to move the slider 152 toward the front end of the slider receiving portion 154. At this time, a frictional force between the slider 152 and the slider receiving portion 154 is smaller than that in a case where the slider 152 is moved to the rear end side.

It is hard for the slider 152 to move, due to the friction between the slider and the slider receiving portion 154, and the slider is regulated so that it takes from several minutes to about five minutes for the second jaw 52 shown in FIG. 5A to reach the state shown in FIG. 5B (reach the closed state from the opened state).

As an example where the frictional force is heightened when the slider 152 moves in one direction and the frictional force is lowered when the slider moves in an opposite direction, for example, a gentle tilt may be applied. That is, each of the slider 152 and the slider receiving portion 154 is preferably shaped in the form of a wedge.

Next, an operation of the treatment device 12 according to this embodiment will be described.

In a state where the second jaw 52 is closed to the first jaw 42 as shown in FIG. 5B, a treatment section 26 is opposed to a biological tissue. In this state, the pressing body 166 is advanced, and the slider 152 is advanced against the urging force of the compression coil spring 158. Further, the slider 152 is supported at a predetermined position by the restricting pin 156. At this time, the second jaw 52 opens from the first jaw 42.

As shown in FIG. 5A, in the state where the second jaw 52 is opened from the first jaw 42, the restricting pin (an operating body) 156, a part of which is disposed in the slider receiving portion 154, is moved to the outside of the slider receiving portion 154. By the urging force of the compression coil spring (the interlocking mechanism) 158, the slider (the interlocking mechanism) 152 moves to a proximal end side and pulls the wire (the interlocking mechanism) 160, whereby a position of the restricting pin 156 moves from upper left to lower right in FIG. 5A. Consequently, the second jaw 52 comes close to the first jaw 42. At this time, it is difficult for the slider 152 to move to the slider receiving portion 154 on the rear end side, due to the friction. In consequence, the first and second jaws 42 and 52 are prevented from being vigorously closed, and take time to be slowly closed.

Consequently, as described in the first embodiment, the first and second jaws 42 and 52 are prevented from being vigorously closed, and take time to be slowly closed. At this time, an operator does not have to continue operating the wire 160 so that the first and second jaws 42 and 52 are prevented from being vigorously closed, and burdens on the operator can noticeably be decreased. In addition, depending on a degree of difficulty of the first and second jaws 42 and 52 moving the slider 152 to the slider receiving portion 154 on the rear end side, the jaws are closed at a substantially constant speed, so that it is possible to inhibit fluctuation of a closing amount per unit time due to operator's power adjustment when the wire 160 is pulled to the handle 22, and unification of quality of a medical treatment can be achieved.

It is to be noted that when the treatment in which energy is used is ended, the pressing body 166 is advanced, and then the slider 152 is advanced against the urging force of the compression coil spring 158. Further, the restricting pin 156 supports the slider 152 at the predetermined position. Consequently, the second jaw 52 can be opened from the first jaw 42.

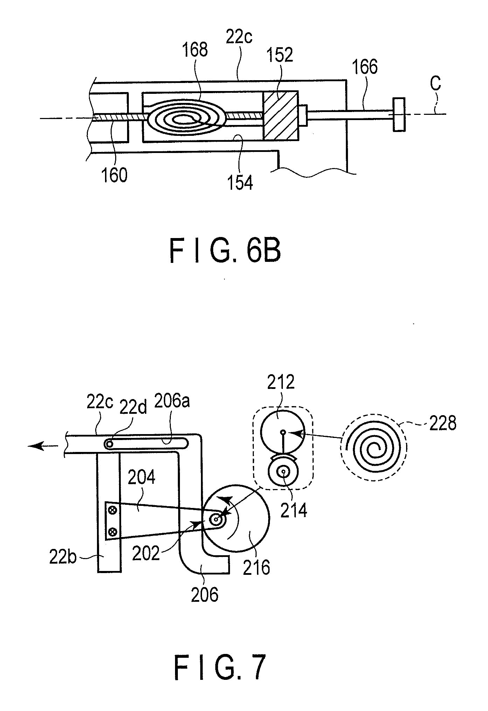

In this embodiment, there has been described an example where the compression coil spring (the interlocking mechanism) 158 is used, but as shown in FIG. 6A and FIG. 6B, a spiral spring (an interlocking mechanism) 168 is preferably used. The spiral spring 168 increases an urging force to urge the slider 152 toward a rear end when the slider 152 is moved to the front side, and the spiral spring minimizes the urging force when the slider 152 is moved to a rear side.

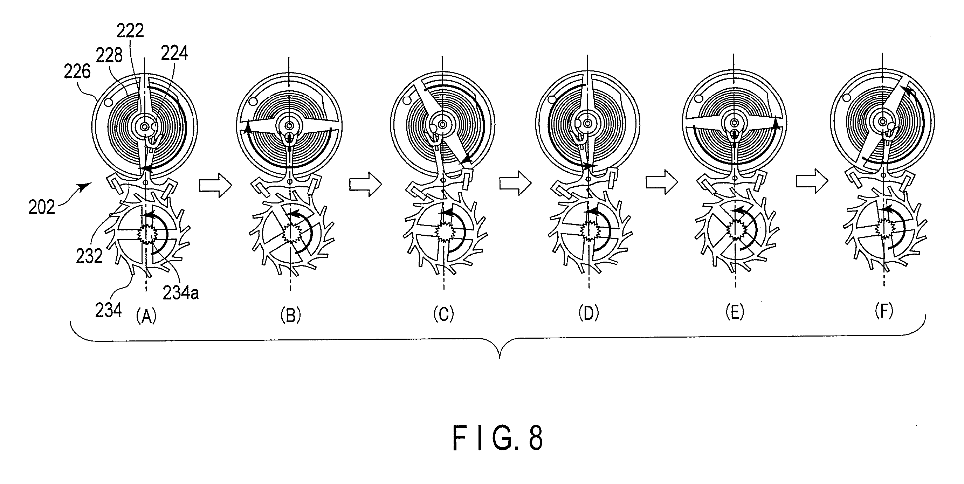

Next, a fourth embodiment will be described with reference to FIG. 7 and FIG. 8. This embodiment is a modification of the first to third embodiments, the same members or members having the same functions as in the first to third embodiments are denoted with the same reference signs, and detailed descriptions of the members are omitted.

In a treatment device 12 according to this embodiment, a fixed plate 204 is extended in which a cam mechanism (an interlocking mechanism) 202 is disposed on a rear side of a handle 22. A movable lever (an operating body) 206 that is movable along a longitudinal axis of a shaft 24 is supported In the handle 22. A long hole 206a into which a pin 22d disposed in the handle 22 is engaged is formed in the movable lever 206. Consequently, the movable lever 206 is formed to only move in a predetermined region.

In this embodiment, the cam mechanism 202 includes a speed regulator (an interlocking mechanism) 212 and an escapement (an interlocking mechanism) 214, that are used in a pocket watch, a wristwatch or the like, at a rear end of the fixed plate 204. The cam mechanism 202 further includes a cam (an interlocking mechanism) 216.

As shown in (A) to (F) of FIG. 8, the speed regulator 212 includes a swing seat 222 that is present at a central position, a swing stone 224 that is disposed in the swing seat 222 and present at an eccentric position, a circular balance wheel 226 around the swing seat 222, and a balance spring 228 that expands and contracts based on the swing seat 222. In the speed regulator 212, the balance wheel 226 regularly repeats a reciprocating rotary motion by the expansion/contraction of the balance spring 228 having isochronism.

The escapement 214 includes an anchor 232 that performs a pendulum motion by the reciprocating motion of the balance wheel 226, and an escape wheel 234 having a gear portion 234a at the center. The anchor 232 performs the pendulum motion to rotate the escape wheel 234, thereby rotating the gear portion 234a. On the other hand, the escape wheel 234 and the anchor 232 continue applying a force for the balance wheel 226 to perform the reciprocating motion. The cam 216 is fixed to the gear portion 234a of the escape wheel 234.

Consequently, as shown in (A) to (C) of FIG. 8, the balance wheel 226 rotates clockwise. Afterward, by the balance spring 228, the balance wheel 226 rotates counterclockwise as shown in (D) to (F) of FIG. 8. It is to be noted that a speed of the balance wheel 226 per unit time can suitably be set.

A force from the balance spring (an interlocking mechanism) 228 is transmitted to the escape wheel (an interlocking mechanism) 234. The anchor (an interlocking mechanism) 232 is moved by a force with which the escape wheel 234 is to rotate. The anchor 232 vibrates (rotates) the balance wheel (an interlocking mechanism) 226. The vibration of the balance wheel 226 works to move the anchor 232 alternately to the right and left. The motion of the anchor 232 feeds teeth of the escape wheel 234 one by one. When the balance wheel 226 reciprocates once, the escape wheel 234 turns as much as the number of the teeth/360 degrees.

Further, the cam 216 is rotated by the gear portion 234a in the center of the escape wheel 234. Consequently, the cam 216 presses the movable lever (the operating body) 206 to advance the lever 206.

Therefore, an outer cylinder 34 advances in accordance with the advancement of the movable lever 206 as a pressing body. Depending on a shape of the cam 216, each of first and second jaws 42 and 52 is closed every substantially suitable amount.

Therefore, as described in the first embodiment, according to the treatment device 12 of this embodiment, the first and second jaws 42 and 52 are prevented from being vigorously closed, and take time to be slowly closed. At this time, an operator does not have to continue operating the movable lever 206 of the handle 22 so that the first and second jaws 42 and 52 are prevented from being vigorously closed, and burdens on the operator can noticeably be decreased. In addition, the first and second jaws 42 and 52 relatively close at a substantially constant speed by the cam mechanism 202 having the speed regulator 212 and the escapement 214, so that it is possible to inhibit fluctuation of a closing amount per unit time due to operator's power adjustment when the movable lever 206 is pressed to the handle 22, and unification of quality of a medical treatment can be achieved.

It is to be noted that when the first and second jaws 42 and 52 are opened, the movable lever 206 may be moved to a rear end side.

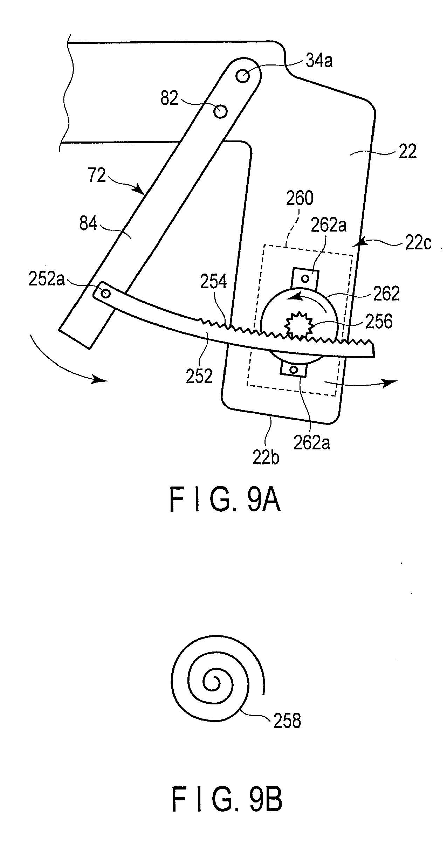

Next, a fifth embodiment will be described with reference to FIG. 9A and FIG. 9B. This embodiment is a modification of the first to fourth embodiments, the same members or members having the same functions as in the first to fourth embodiments are denoted with the same reference signs, and detailed descriptions of the members are omitted.

As shown in FIG. 9A, a supporter 252 having a rack (an interlocking mechanism) 254 is supported between an opening/closing lever (an operating body) 72 and the other end 22b of a handle 22. The supporter 252 is pivotally supported by a pivot support shaft 252a. At the other end 22b of the handle 22, a pinion (an interlocking mechanism) 256 meshed with the rack 254 is rotatably supported by an elastic member (an interlocking mechanism) 258 such as a spiral spring shown in FIG. 9B. The spiral spring 258 can be supported in a state where the pinion 256 is urged in accordance with a position of the opening/closing lever 72. That is, when the opening/closing lever 72 is separated from the other end 22b of the handle 22, the spiral spring 258 exerts an urging force on the pinion 256. In other words, the spiral spring (the elastic member) 258 of the pinion 256 exerts the urging force to urge the opening/closing lever 72 toward a handle main body 22c, between the opening/closing lever (the operating body) 72 and the handle main body 22c. On the other hand, when the opening/closing lever 72 is brought close to the other end 22b of the handle 22, the spiral spring 258 does not exert the urging force on the pinion 256.

Around the pinion 256 and the spiral spring 258, a damper 260 including the rack 254 and containing, for example, oil is disposed. The damper 260 buffers the urging force between the opening/closing lever 72 and the handle main body 22c, and regulates a closing time until first and second jaws 42 and 52 reach a closed state from an opened state. The damper 260 includes a friction plate 262 having a blade 262a. It is to be noted that the pinion 256 and the spiral spring 258 are present on the same axis, and the friction plate 262 having the blade 262a is disposed in the pinion 256 and the spiral spring 258. Oil is present around the friction plate 262, and hence, rotation of the pinion 256 by the spiral spring 258 is controlled by the friction plate 262. For example, ease of rotating the pinion 256 is determined by a shape or size of the blade 262a or a shape or size of the friction plate 262 itself. In addition, a type of oil or the like is suitably set to regulate viscosity, so that a rotation amount of the pinion 256 per unit time can be regulated.

An operator separates the opening/closing lever 72 from the other end 22b of the handle 22, and the urging force is gradually applied to the spiral spring 258. At this time, the second jaw 52 opens from the first jaw 42. When the operator releases the opening/closing lever 72 in this state, the pinion 256 rotates in a direction shown in FIG. 9A by the urging force of the spiral spring 258. Consequently, the opening/closing lever 72 comes close to the other end 22b of the handle 22 by the rack 254. At this time, the oil damper 260 having the friction plate 262 can inhibit rapid rotation of the pinion 256.

Therefore, as the opening/closing lever 72 comes close to the other end 22b of the handle 22, an outer cylinder 34 advances, and each of the first and second jaws 42 and 52 is closed every substantially suitable amount.

Therefore, as described in the first embodiment, according to a treatment device 12 of this embodiment, the first and second jaws 42 and 52 are prevented from being vigorously closed, and take time to be slowly closed. At this time, the operator does not have to continue operating the opening/closing lever 72 of the handle 22 so that the first and second jaws 42 and 52 are prevented from being vigorously closed, and burdens on the operator can noticeably be decreased. In addition, the first and second jaws 42 and 52 are relatively closed at a substantially constant speed, and hence, it is possible to inhibit fluctuation of a closing amount per unit time due to operator's power adjustment when the opening/closing lever 72 is pressed to the handle 22, and unification of quality of a medical treatment can be achieved.

It is to be noted that, when the first and second jaws 42 and 52 are opened, the opening/closing lever 72 may be separated from the other end 22b of the handle 22.

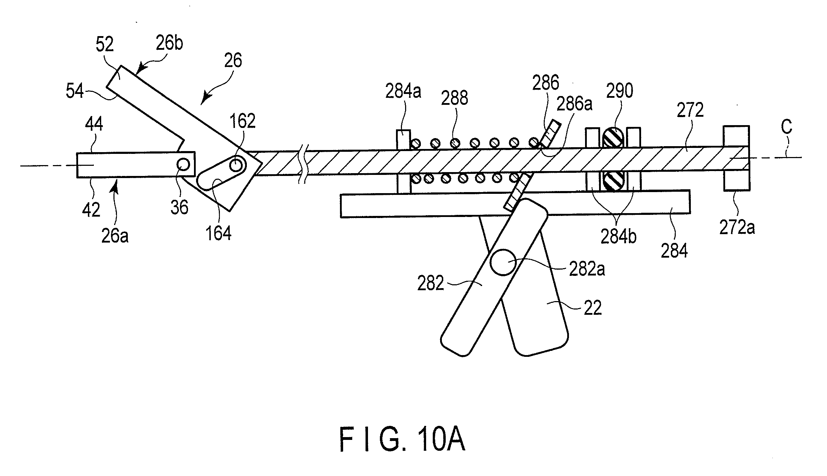

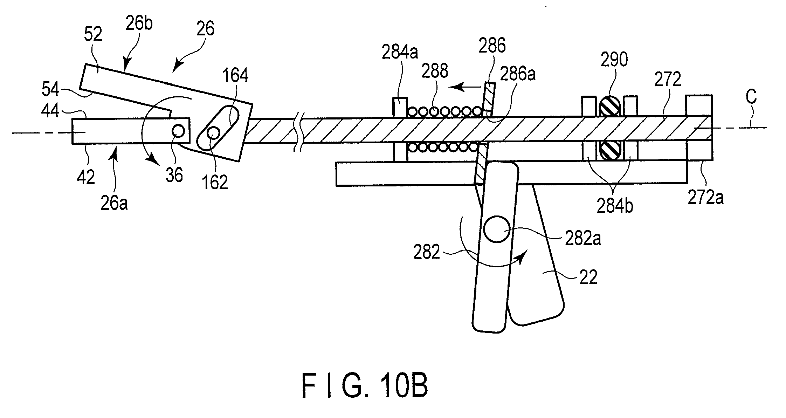

Next, a sixth embodiment will be described with reference to FIG. 10A and FIG. 10B. This embodiment is a modification of the first to fifth embodiments, the same members or members having the same functions as in the first to fifth embodiments are denoted with the same reference signs, and detailed descriptions of the members are omitted.

As shown in FIG. 10A and FIG. 10B, a treatment device 12 according to this embodiment includes a wire-like, rod-like or tubular movable member (an interlocking mechanism) 272 that is movable along an axial direction.

In a handle 22, an opening/closing lever (an operating body) 282 is pivotally supported by a pivot support portion 282a. Further in the handle 22, there are disposed an extending portion (an interlocking mechanism) 284 extended in parallel with, for example, the movable member 272, a lock plate (an interlocking mechanism) 286 as a pressing body that restricts movement of the movable member 272, an elastic member (an interlocking mechanism) 288 such as a compression coil spring that urges the lock plate 286 toward a rear end side, and an O-ring (an interlocking mechanism) 290 disposed in an outer periphery of the movable member 272 and supported to the extending portion 284. The O-ring 290 is restricted from moving in an axial direction of the extending portion 284, and applies a frictional force between the ring and an outer peripheral surface of the movable member 272.

In the extending portion 284, there is disposed the lock plate 286 having a hole portion 286a into which the movable member 272 can be inserted. In the extending portion 284, there is disposed a support plate 284a that supports one end of the compression coil spring 288. The other end of the compression coil spring 288 is supported by the lock plate 286. The lock plate 286 is supported by the opening/closing lever 282. In the extending portion 284, there are formed sandwiching portions 284b that restrict movement of the O-ring 290 in a forward-backward direction.

As shown in FIG. 10A, the movement of the movable member 272 is restricted by friction between the outer peripheral surface of the movable member 272 and the hole portion 286a of the lock plate 286. When the lever 282 is brought close to the other end of the handle 22 against an urging force of the compression coil spring 288, the movable member 272 is advanced by the friction between the outer peripheral surface of the movable member 272 and the hole portion 286a of the lock plate 286 as shown in FIG. 10B.

It is to be noted that against a frictional force between the O-ring 290 and the outer peripheral surface of the movable member 272, the movable member 272 can be advanced by operating the opening/closing lever 282, but the movable member 272 advances to such an extent that the movement of the movable member is stopped to the O-ring 290 in a state where the opening/closing lever 282 is relatively stopped to the handle 22.

It is to be noted that it is hard for the movable member 272 to move, due to the frictional force between the member and the O-ring 290. That is, the O-ring 290 functions as a restricting portion that restricts the movable member 272 from retreating. Consequently, the movable member 272 slowly advances to the O-ring 290.

Further, when the opening/closing lever 282 is opened, the lock plate 286 returns to its original position by the urging force of the compression coil spring 288. On the other hand, the O-ring 290 applies a brake to the movement of the movable member 272 by the frictional force between the ring and the outer peripheral surface of the movable member 272. Consequently, in a state where jaws 42 and 52 are closed as shown in FIG. 10B, the only opening/closing lever 282 can be returned to the state shown in FIG. 10A.

Therefore, the opening/closing lever 282 is brought close to or separated from the handle 22, whereby the movable member 272 gradually advances to relatively close the jaws 42 and 52. That is, the movable member (the interlocking mechanism) 272 has a constitution where every time an operation of bringing the opening/closing lever (the operating body) 282 close to the handle main body 22c and separating the lever from the main body is input as one cycle, an opened state of the first and second jaws 42 and 52 is brought close to the closed state.

Here, a closing amount of the jaws 42 and 52 per grip of the opening/closing lever 282, i.e., a unit closing amount per one operation becomes constant. Consequently, it is possible to inhibit fluctuation of the closing amount due to operator's power adjustment.

When operations of cycles are input into the opening/closing lever 282 for the handle 22, it is possible to regulate the closing amount (the unit closing amount) per cycle until the first and second jaws 42 and 52 reach the closed state from the opened state.

It is to be noted that when the state where the second jaw 52 is closed to the first jaw 42 as shown in FIG. 10B shifts to such an opened state as shown in FIG. 10A, the opening/closing lever 282 is closed to the handle 22. Consequently, a frictional force between the movable member 272 and the hole portion 286a of the lock plate 286 becomes smaller. In this state, a proximal end 272a of the movable member 272 is pulled to the rear end side against the frictional force of the O-ring 290. Therefore, the second jaw 52 can easily be opened from the first jaw 42.

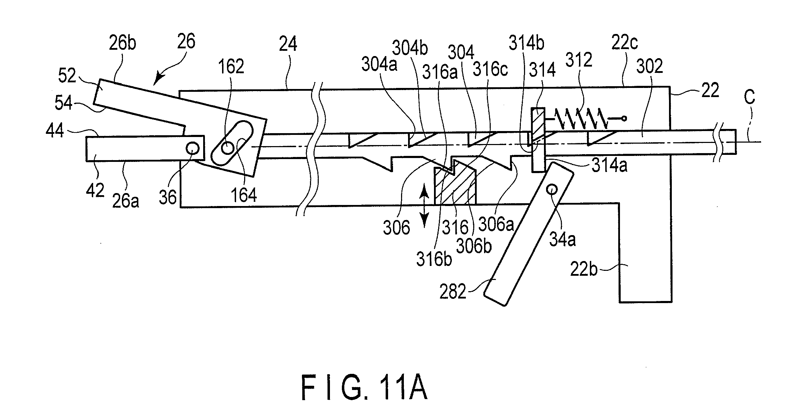

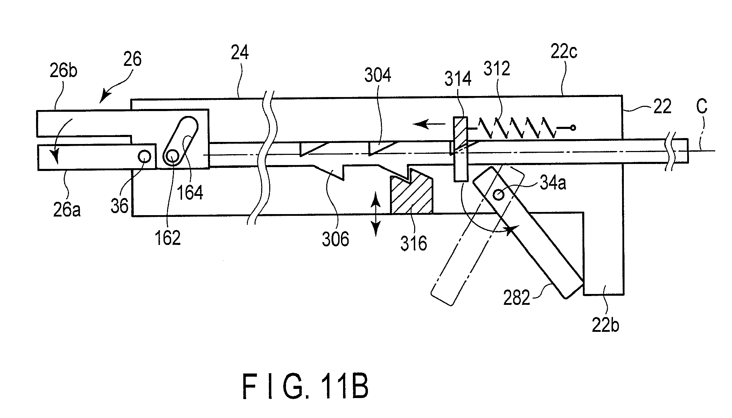

Next, a seventh embodiment will be described with reference to FIG. 11A and FIG. 11B. This embodiment is a modification of the first to sixth embodiments, the same members or members having the same functions as in the first to sixth embodiments are denoted with the same reference signs, and detailed descriptions of the members are omitted.

As shown in FIG. 11A and FIG. 11B, a treatment device 12 according to this embodiment includes a movable rod (an interlocking mechanism) 302 movable along an axial direction.

The movable rod 302 movable along the axial direction includes groove portions 304 along the axial direction and projections 306 along the axial direction. The groove portions 304 and the projections 306 are arranged at predetermined intervals, respectively.

All the groove portions 304 are disposed on upper sides of the FIG. 11A and FIG. 11B, and all the projections 306 are disposed on lower sides of the FIG. 11A and FIG. 11B. That is, the groove portions 304 and the projections 306 are formed at mutually opposite positions to a central axis of the movable rod 302. Each of the groove portions 304 includes an orthogonal surface 304a whose distal end side is deeply cut from an outer peripheral surface and which is orthogonal to a central axis C, and an inclined surface 304b that comes close to the outer peripheral surface from a deep portion of the orthogonal surface 304a to the outer peripheral surface toward a proximal end side. Each of the projections 306 includes an orthogonal surface 306a that projects from the outer peripheral surface on the proximal end side and is orthogonal to the central axis C, and an inclined surface 306b that comes close to the outer peripheral surface from a projecting portion of the orthogonal surface 306a to the outer peripheral surface toward the distal end side.

In a handle 22, one end (a proximal end) of an elastic member 312 such as a tensile coil spring is supported, and the other end (a distal end) of the tensile coil spring 312 supports an operating plate (an interlocking mechanism) 314. The operating plate 314 is pulled toward a proximal end of the handle 22 by the tensile coil spring 312. The operating plate 314 includes a pressing force receiving portion 314a to which a pressing force is applied by an operation of an opening/closing lever 282, and an inclined surface 314b along the inclined surface 304b of the groove portion 304.

In a shaft 24 or the handle 22, an engaging portion (an interlocking mechanism) 316 that engages with the projection 306 is disposed. The engaging portion 316 includes an inclined surface 316a along the inclined surface 306b of the projection 306, and an orthogonal surface 316b facing the orthogonal surface 306a of the projection 306, and the engaging portion engages with the projection 306. The engaging portion 316 includes a proximal end side inclined surface 316c on which the projection 306 is slid. It is to be noted that the engaging portion 316 is engageable with and disengageable from the movable rod 302, and the engaging portion is engaged when jaws 42 and 52 are closed, and is disengaged when the jaws 42 and 52 are opened. When the engaging portion 316 is engaged to close the jaws 42 and 52, the engaging portion functions as a restricting portion that restricts the movable rod 302 from retreating.

When the opening/closing lever 282 is brought close to the other end 22b of the handle 22 in a state shown in FIG. 11A, the operating plate 314 advances against an urging force of the tensile coil spring 312 as shown in FIG. 11B. At this time, the operating plate 314 presses the orthogonal surface 304a of the groove portion 304. Consequently, the movable rod 302 advances.

With the advancement of the movable rod 302, the projection 306 rides across the proximal end side inclined surface 316c of the engaging portion 316. Consequently, the projection 306 is engaged with the engaging portion 316. On the other hand, by a tensile force of the tensile coil spring 312, the operating plate 314 slides on the inclined surface 304b of one of the groove portions 304 and the outer peripheral surface to be disposed in the groove portion 304 on the proximal end side from the one groove portion 304. At this time, as shown in FIG. 11A, the pressing force receiving portion 314a of the operating plate 314 is supported by the opening/closing lever 282.

Such an operation (cycle) is repeated, and the movable rod 302 is gradually advanced. Consequently, the second jaw 52 can slowly and gradually be closed to the first jaw 42 every predetermined amount.

It is to be noted that when the second jaw 52 is opened from the first jaw 42, the engaging portion 316 is separated from the movable rod 302. Consequently, when the movable rod 302 is moved to the proximal end side, the engaging portion is prevented from being engaged.

Therefore, the opening/closing lever 282 is brought close to and separated from the handle 22, whereby the jaws 42 and 52 are gradually and relatively closed. Here, a closing amount of the jaws 42 and 52 per grip of the opening/closing lever 282 (a unit closing amount per one operation) substantially becomes constant. Consequently, it is possible to inhibit fluctuation of the closing amount due to operator's power adjustment.

When operations of cycles are input into the opening/closing lever 282 for the handle 22, it is possible to regulate the closing amount (the unit closing amount) per cycle until the first and second jaws 42 and 52 reach a closed state from an opened state.

When the second jaw 52 is opened from the first jaw 42, the engaging portion 316 is retracted from the handle 22. Further, a proximal end of the movable rod 302 is pulled to the proximal end side. Consequently, the movable rod 302 gradually moves to a rear end side. In consequence, the second jaw 52 opens from the first jaw 42. Further, the operating plate 314 engages with the foremost groove portion 304. Further, every time the opening/closing lever 282 is operated, the operating plate 314 is engaged with the rear side groove portions 304 one by one.

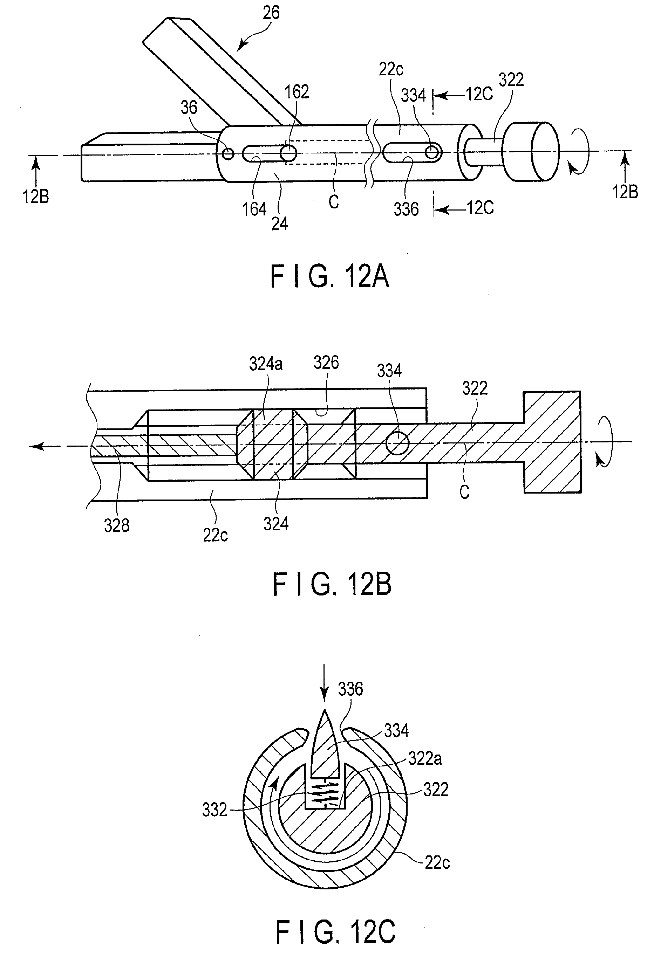

Next, an eighth embodiment will be described with reference to FIG. 12A to FIG. 12C. This embodiment is a modification of the first to seventh embodiments, the same members or members having the same functions as in the first to seventh embodiments are denoted with the same reference signs, and detailed descriptions of the members are omitted.

As shown in FIG. 12A to FIG. 12C, a treatment device 12 according to this embodiment includes a rotary rod (an operating body) 322 that rotates in a periaxial direction of a central axis C to be movable along an axial direction. A large diameter portion 324 having an external thread portion 324a is disposed at a distal end of the rotary rod 322. The large diameter portion 324 has an outer diameter larger than an outer diameter of the rotary rod 322. It is to be noted that, in a handle 22 (on an inner side), there is formed an internal thread portion 326 into which the external thread portion 324a of the large diameter portion 324 can be screwed.

At a further distal end of the large diameter portion 324 of a distal end of the rotary rod 322, there is supported a movable rod 328 that moves in the axial direction in a state where rotation is restricted as the rotary rod 322 moves in the axial direction. The large diameter portion 324 and the movable rod 328 are formed as in a known ball screw structure.

As shown in FIG. 12C, a concave portion 322a is formed in a direction orthogonal to the axial direction in the rotary rod 322. In the concave portion 322a, a convex portion (a projecting body) 334 is disposed via an elastic member (an interlocking mechanism) 332 such as a compression coil spring. The convex portion 334 is formed to be tapered from an inner side of the rotary rod 322 in a radial direction toward an outer side of the rotary rod in the radial direction. Further, the convex portion 334 projects to the outside of a handle main body 22c in the radial direction by an urging force of the compression coil spring 332.

In the main body 22c of the handle 22, for example, a long hole (an interlocking mechanism) 336 long in the axial direction is formed so that the convex portion 334 is urged to project to the outside of the rotary rod 322 in the radial direction. Every time the rotary rod 322 is rotated once in the periaxial direction of the central axis C, the convex portion 334 projects to the long hole 336 by an operation of the compression coil spring 332. Here, the convex portion 334 is formed to be tapered from the inner side of the radial direction toward the outer side of the radial direction, and hence, the convex portion surely projects to the outside of the long hole 336 in the radial direction every time the rotary rod 322 is rotated once in the periaxial direction of the central axis C. Further, the convex portion 334 moves every predetermined pitch toward the distal end in the axial direction every time the rotary rod 322 is rotated once (one cycle) in a predetermined direction (the periaxial direction of the central axis C).

Consequently, the convex portion 334 can be projected from the long hole 336 every time the rotary rod 322 is rotated once to the handle 22 in the periaxial direction of the central axis C. Further, the rotary rod 322 is rotated while pressing the convex portion 334 of the rotary rod, so that it is possible to further rotate the rotary rod 322 once.

When the rotary rod 322 is rotated once (one cycle) in the periaxial direction of the central axis C, a moving amount of the movable rod 328 is a predetermined amount. Therefore, when an operation of one cycle (one rotation) is input into the rotary rod 322, the movable rod 328 advances as much as the predetermined amount. That is, the movable rod (an interlocking mechanism) 328 has a constitution where an opened state of first and second jaws 42 and 52 is brought close to a closed state every time an operation of allowing the rotary rod (the operating body) 322 to rotate once in the predetermined direction to the handle main body 22c is input as one cycle. Consequently, it is possible to regulate a closing amount (a unit closing amount per one operation) when the operation of the one cycle (one rotation) is input into the rotary rod 322. That is, cycles (rotations) are required until the first and second jaws 42 and 52 reach the closed state from the opened state, but it is possible to regulate the number of the cycles required until the closed state is reached from the opened state.

It is to be noted, when the second jaw 52 is set to the opened state from the closed state to the first jaw 42, the rotary rod 322 is reversely rotated in the periaxial direction of the central axis C, whereby the movable rod 328 may be moved to a proximal end side. At this time, the convex portion 334 and the long hole 336 function as a restricting portion that restricts the movable rod 328 from retreating.

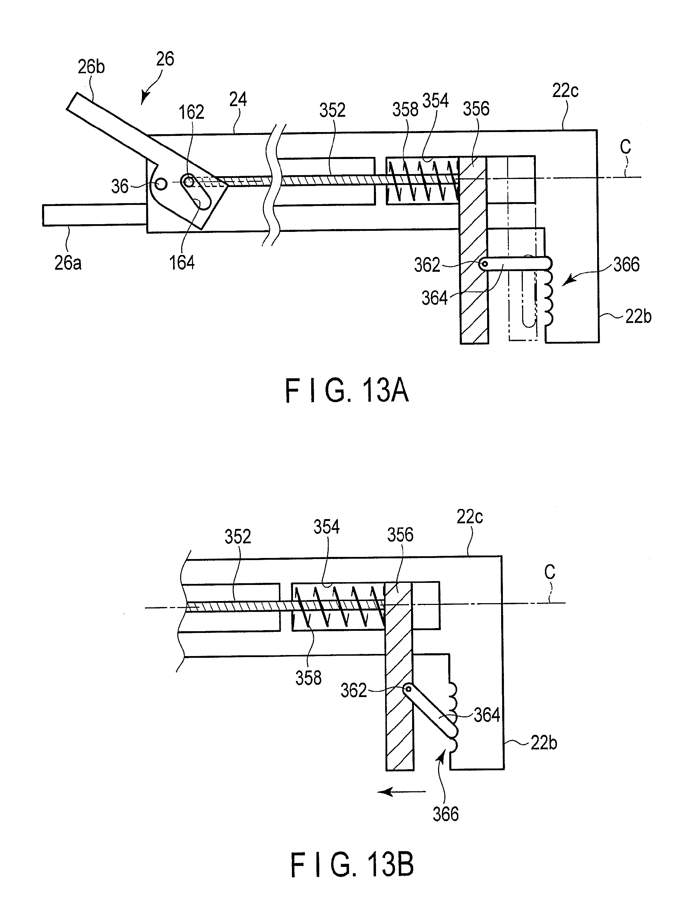

Next, a ninth embodiment will be described with reference to FIG. 13A and FIG. 13B. This embodiment is a modification of the first to eighth embodiments, the same members or members having the same functions as in the first to eighth embodiments are denoted with the same reference signs, and detailed descriptions of the members are omitted.

As shown in FIG. 13A and FIG. 13B, a handle 22 of a treatment device 12 according to this embodiment is coupled with first and second jaws 42 and 52 by a wire (an interlocking mechanism) 352. When the wire 352 is pulled, the second jaw 52 can gradually be closed to the first jaw 42.

A receiving chamber (an interlocking mechanism) 354 is formed in the handle 22. In the receiving chamber 354, there is disposed a movable body (an interlocking mechanism) 356 movable along an axial direction of the wire 352. The movable body 356 is extended from a handle main body 22c so that the movable body is disposed in parallel with the other end 22b of the handle 22.

The wire 352 is inserted into the receiving chamber 354, and a proximal end of the wire 352 is fixed to the movable body 356. Between the movable body 356 and a front end of the receiving chamber 354, an elastic member (an interlocking mechanism) 358 such as a compression coil spring is supported along an outer periphery of the wire 352.

As shown in FIG. 13A, in this embodiment, the movable body is formed so that the second jaw 52 is closed to the first jaw 42 by pulling the movable body 356 toward a rear end of the wire 352 in the axial direction.

As shown in FIG. 13A and FIG. 13B, one end of a rotary rod (an operating body) 364 pivotally supported by a rotary shaft 362 is supported in the movable body 356. In the other end 22b of the handle 22, there are formed engaging portions (an interlocking mechanism) 366 to support the other end of the rotary rod 364.

Consequently, as shown in FIG. 13A, the second jaw 52 opens most from the first jaw 42 when the rotary rod 364 is disposed horizontally or substantially horizontally with the wire 352. As the rotary rod 364 tilts to the wire 352 and comes close in an extending direction of the movable body 356, a pulling amount of the wire 352 to be pulled to a rear end side increases, and hence, the second jaw 52 is closed to the first jaw 42.

At this time, the other end of the rotary rod 364 is successively supported by the engaging portions 366 of the other end 22b of the handle 22 in order from a position close to the wire 352 toward a position separated from the wire. Consequently, the second jaw 52 is gradually closed to the first jaw 42.

Therefore, the rotary rod 364 is gradually shifted from the horizontal state to the inclined state to the wire 352, whereby the jaws 42 and 52 are gradually and relatively closed. Here, a closing amount (a unit closing amount per one operation) of the jaws 42 and 52 per cycle in which engaging positions of the rotary rod 364 are shifted one by one against an urging force of the elastic member 358 substantially becomes constant. Consequently, it is possible to inhibit fluctuation of the closing amount due to operator's power adjustment.

When operations of cycles are input into the rotary rod 364 for the handle 22, it is possible to regulate the closing amount (the unit closing amount) for each cycle until the first and second jaws 42 and 52 reach a closed state from an opened state. Further, the operator can suitably set a time to move the rotary rod 364. In consequence, it is possible to regulate the time until the closed state is reached from the opened state.

It is to be noted that, when the second jaw 52 opens from the first jaw 42, the movable body 356 may be advanced against the urging force of the elastic member 358.

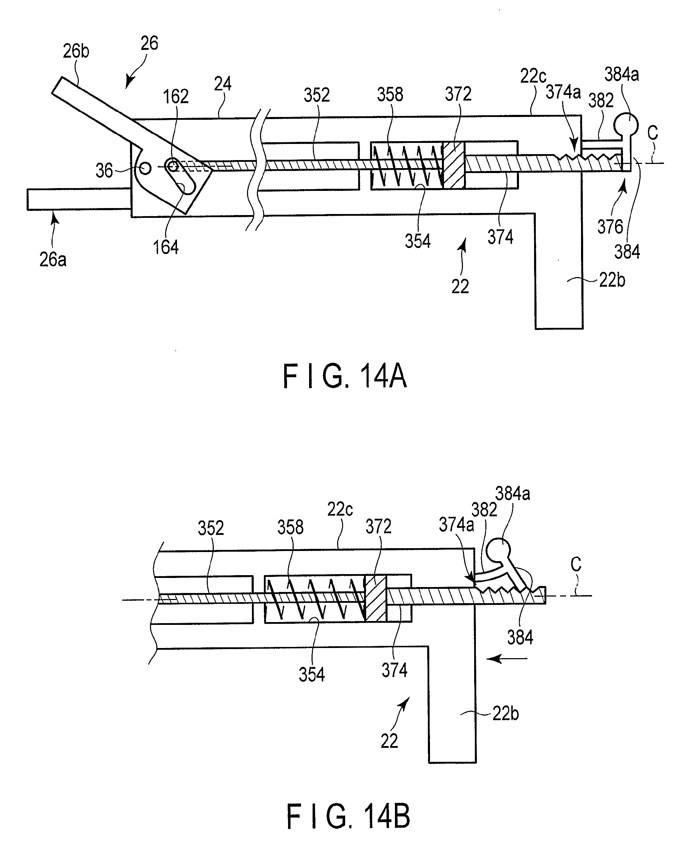

Next, a tenth embodiment will be described with reference to FIG. 14A and FIG. 14B. This embodiment is a modification of the first to ninth embodiments, the same members or members having the same functions as in the first to ninth embodiments are denoted with the same reference signs, and detailed descriptions of the members are omitted. This embodiment is especially a modification of the ninth embodiment.

In a receiving chamber (an interlocking mechanism) 354 of a handle 22 of a treatment device 12 according to this embodiment, a slider (an interlocking mechanism) 372 is supported at a proximal end of an elastic member (an interlocking mechanism) 358 such as a compression coil spring. An engaging rod (an operating body) 374 extended from a proximal end of the handle 22 is fixed to the slider 372. The engaging rod 374 includes engaging portions (an interlocking mechanism) 374a.

As shown in FIG. 14A and FIG. 14B, an engaging portion (an interlocking mechanism) 376 is fixed to the proximal end of the handle 22. The engaging portion 376 includes an extending portion 382 having flexibility, and a rigid pin 384 which has a knob 384a at one end and whose other end is engaged with the engaging portion 374a of the engaging rod 374. It is to be noted that the extending portion 382 is prevented from being extended in an extending direction, but is formed to be bent in a predetermined range.

It is to be noted that, as shown in FIG. 14A, the rigid pin 384 prevents retreating of the engaging rod 374.

By operating the knob 384a, the engaging rod 374 can be retreated by an urging force of the elastic member 358. When the rigid pin 384 is engaged on a rear end side in the engaging portions 374a of the engaging rod 374, a second jaw 52 in an opened state closes toward a first jaw 42. A closing amount at this time depends on a distance from a rear end of the engaging rod 374. The hard pin 384 is engaged with the engaging portions 374a of the engaging rod 374, for example, one by one on a distal end side, thereby releasing the engagement. Consequently, the second jaw 52 closes toward the first jaw 42 in accordance with the distance from the rear end of the engaging rod 374.

Therefore, the engaging rod 374 is retreated by the urging force of the elastic member 358, i.e., the wire 352 is pulled to the rear end side, whereby the jaws 42 and 52 are gradually and relatively closed. Here, a closing amount (a unit closing amount per one operation) of the jaws 42 and 52 per cycle in which positions to engage the engaging portions 374a of the engaging rod 374 with the rigid pin 384 are shifted one by one on the rear end side of the engaging portion 374a of the engaging rod 374 to the rigid pin 384 substantially becomes constant. In consequence, it is possible to inhibit fluctuation of the closing amount due to operator's power adjustment.

When operations of cycles are input into the engaging rod 374 for the handle 22, it is possible to regulate the closing amount (the unit closing amount) per cycle until the first and second jaws 42 and 52 reach a closed state from an opened state. Further, the operator can suitably set a time to move the engaging rod 374. In consequence, it is possible to regulate the time until the closed state is reached from the opened state.

It is to be noted that, when the second jaw 52 opens from the first jaw 42, the engaging rod 374 is pressed toward the distal end side against the urging force of the elastic member 358, i.e., the slider 372 may be advanced against the urging force of the elastic member 358.

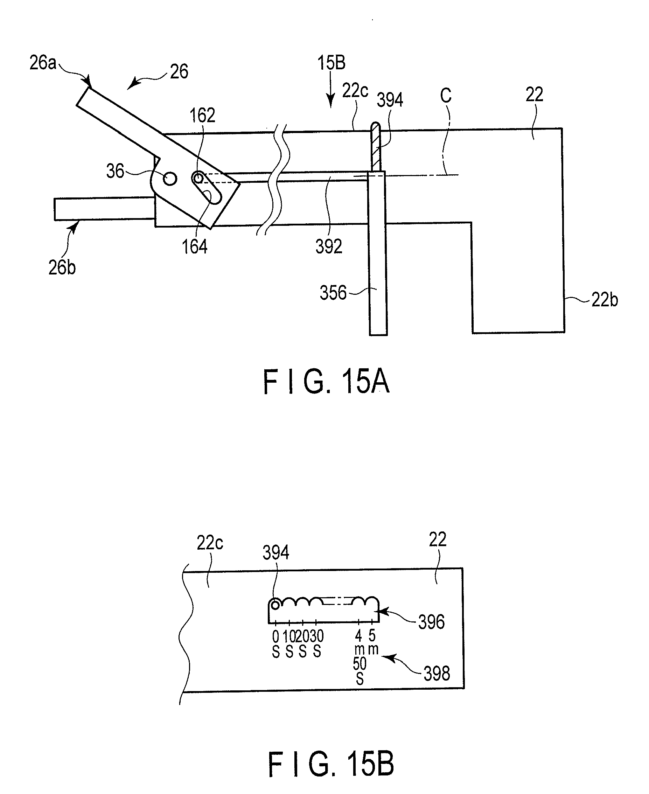

Next, an eleventh embodiment will be described with reference to FIG. 15A to FIG. 16B. This embodiment is a modification of the first to tenth embodiments, the same members or members having the same functions as in the first to tenth embodiments are denoted with the same reference signs, and detailed descriptions of the members are omitted. This embodiment is a modification of the ninth and tenth embodiments.

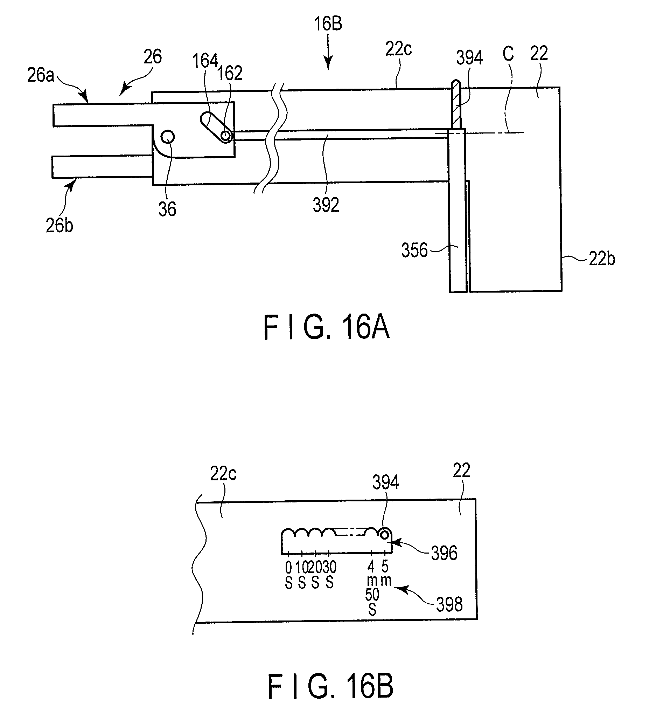

As shown in FIG. 15A and FIG. 16A, in this embodiment, a movable rod (an interlocking mechanism) 392 is used in place of the wire 352. A movable body (an interlocking mechanism) 356 can be moved in parallel with the movable rod 392. An engaging pin 394 extended in an opposite direction to the other end 22b of a handle 22 is fixed to the movable body 356.

As shown in FIG. 15B and FIG. 16B, at one end 22a of the handle 22, the engaging pin 394 is movable in an axial direction of the movable rod 392, and engaging grooves (an interlocking mechanism) 396 are formed at equal intervals in a direction orthogonal to the axial direction of the movable rod 392. When the engaging pin 394 is fitted into the engaging groove 396, the engaging pin is suitably engaged by press-in or the like. When the engaging pin 394 is disengaged from the engaging groove 396, the engaging pin 394 may be pulled out from the engaging groove 396.

As shown in FIG. 15B and FIG. 16B, the engaging pin 394 can be engaged with the engaging groove 396 at a suitable position. At this time, the engaging groove 396 holds the movable rod 392 to restrict the rod from moving in the axial direction.

In the vicinities of the engaging grooves 396, there is described a minimum time (seconds) scale 398 from an opened state as time 0 (seconds) to the respective engaging grooves 396. Consequently, an operator compares the scale 398 with an actual time as to a time at which a second jaw 52 starts to be closed to a first jaw 42, and the operator moves the movable body 356 toward a proximal end of the handle 22 while repeating engagement with and disengagement from the engaging grooves 396. That is, the movable body 356 and the engaging pin 394 are moved in accordance with indications of the scale 398.

Therefore, the movable body 356 shown in FIG. 15A and FIG. 15B are moved to the engaging grooves 396 in order from a distal end side of a handle main body 22c to a proximal end side shown in FIG. 16A and FIG. 16B, whereby the jaws 42 and 52 are gradually and relatively closed. Here, a closing amount (a unit closing amount per one operation) of the jaws 42 and 52 per cycle in which positions to engage the engaging pin 394 with the engaging grooves 396 are shifted one by one on a rear end side substantially becomes constant. In consequence, it is possible to inhibit fluctuation of the closing amount (the unit closing amount) due to operator's power adjustment.

When operations of cycles are input into the movable body 356 for the handle 22, it is possible to regulate the closing amount (the unit closing amount) for each cycle until the first and second jaws 42 and 52 reach a closed state from the opened state. Further, the operator can suitably set a time to move the movable body 356. In consequence, it is possible to regulate the time until the closed state is reached from the opened state.

It is to be noted that, when the second jaw 52 opens from the first jaw 42, the engaging pin 394 is moved to the distal end side without being engaged with the engaging grooves 396, i.e., the movable body 356 may be advanced.

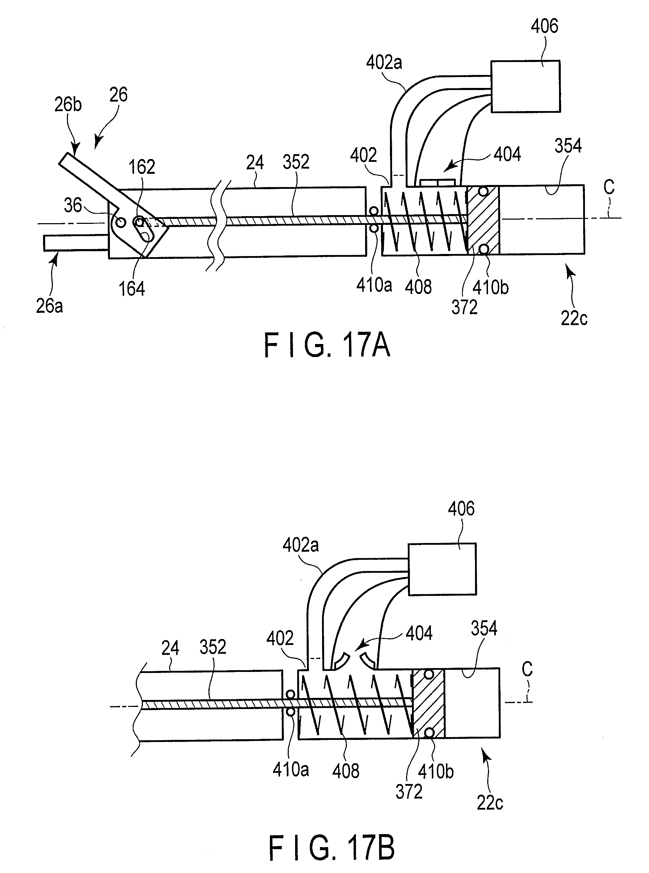

Next, a twelfth embodiment will be described with reference to FIG. 17A and FIG. 17B. This embodiment is a modification of the first to eleventh embodiments, the same members or members having the same functions as in the first to eleventh embodiments are denoted with the same reference signs, and detailed descriptions of the members are omitted.

As shown in FIG. 17A, a treatment device 12 according to this embodiment includes a receiving chamber (an interlocking mechanism) 354 formed in a handle 22, a mouthpiece 402 disposed in the receiving chamber 354 and allowing an inner portion of the receiving chamber 354 to communicate with an outer portion, and a pressure relief valve (an interlocking mechanism) 404 disposed in the receiving chamber 354. The mouthpiece 402 is coupled with one end of a hose 402a. The other end of the hose 402a is coupled with a fluid discharging portion (an operating body) 406 that allows water, air, oil or the like to flow into the receiving chamber 354 through the hose 402a. Consequently, when a pressure in excess of a predetermined pressure is applied to the pressure relief valve 404, the valve is opened, thereby discharging the fluid.

A wire 352 is inserted into the receiving chamber 354, and a slider 372 is disposed at a proximal end of the wire 352. Around the wire 352, there is disposed an elastic member (an interlocking mechanism) 408 such as a tensile coil spring to urge the slider 372 on a distal end side. It is to be noted that O-rings (an interlocking mechanism) 410a and 410b are disposed at a front end of the receiving chamber 354 and an outer peripheral surface of the slider 372.

Consequently, when a fluid is allowed to flow inside through the hose 402a and the mouthpiece 402 from the fluid discharging portion 406, the slider 372 moves to a proximal end side against an urging force of the elastic member 408. Consequently, this movement amount is regulated, and hence, a closing amount of a second jaw 52 to a first jaw 42 can be regulated. Here, a closing amount per unit time is regulated by especially using a flow rate of the fluid to be supplied into the receiving chamber 354.

In consequence, the fluid discharging portion 406 is controlled, whereby as described in the first embodiment, the first and second jaws 42 and 52 are prevented from being vigorously closed, and take time to be slowly closed. At this time, the operator does not have to continue operating the movable body 356 of the handle 22 so that the first and second jaws 42 and 52 are prevented from being vigorously closed, and burdens on the operator can noticeably be decreased. In addition, the first and second jaws 42 and 52 are relatively closed at a substantially constant speed by the elastic member 134, so that it is possible to inhibit fluctuation of a closing amount per unit time due to operator's power adjustment when the rotary rod 122 is rotated to the handle 22, and unification of quality of a medical treatment can be achieved.

It is to be noted that the relief valve 404 is opened, whereby the slider 372 can be moved to the distal end side by the tensile coil spring 408, so that the second jaw 52 can be opened from the first jaw 42.

Hitherto, several embodiments have specifically been described with reference to the drawings, but this invention is not limited to the abovementioned embodiments, and includes all implementations to be performed without departing from the scope of the invention.

## Claims

1. A treatment device which applies energy to a biological tissue to treat the biological tissue, comprising:
first and second jaws that are closable to and separable from each other;
a first holding surface that is disposed on the first jaw;
a second holding surface that is disposed on the second jaw to face the first holding surface, and is relatively openable and closable between an opened state where the second holding surface is separated from the first holding surface and a closed state where the second holding surface is close to the first holding surface;
an energy output portion that is disposed in at least one of the first and second holding surfaces, and outputs the energy to the biological tissue held between the first holding surface and the second holding surface;
a handle that is supported by an operator;
an operating body that is disposed in the handle and into which an operation of bringing the first and second jaws close to each other is inputtable; and
an interlocking mechanism that regulates at least one of a closing time until the first and second jaws reach the closed state from the opened state when one operation is input into the operating body for the handle, and a unit closing amount from the opened state toward the closed state, that interlocks at least one of the first and second jaws with the operating body, and that is interposed between the operating body and the handle.

2. The treatment device according to claim 1, wherein the handle includes a handle main body, and
the interlocking mechanism includes an elastic member that couples the operating body with the handle main body, pulls the operating body toward the handle main body at a constant load or a constant pressure, and regulates the closing time until the closed state is reached from the opened state.

3. The treatment device according to claim 1, wherein the handle includes a handle main body, and
the interlocking mechanism includes an elastic member that exerts an urging force to urge the operating body toward the handle main body between the operating body and the handle main body,
the treatment device comprising a damper that buffers the urging force and regulates the closing time until the first and second jaws reach the closed state from the opened state, between the operating body and the handle main body.

4. The treatment device according to claim 3, wherein the interlocking mechanism includes a pinion that is disposed in the handle main body and rotates by the urging force of the elastic member, and a rack meshed with the pinion and supported by the operating body.

5. The treatment tool according to claim 1, wherein the interlocking mechanism includes an elastic member that urges the first and second jaws to close the first and second jaws until the closed state is reached from the opened state, a cam that is disposed in the elastic member and regulates the closing time until the closed state is reached from the opened state, and a pressing body that is moved in accordance with a motion of the cam and operates the first and second jaws to close the first and second jaws.

6. The treatment device according to claim 1, wherein the operating body includes a rotary rod having an external thread, and
the interlocking mechanism includes an internal thread into which the external thread is screwed in the handle, and an axially movable body that operates the first and second jaws to close the first and second jaws, and is configured to regulate the closing time until the closed state is reached from the opened state.

7. The treatment device according to claim 1, wherein the interlocking mechanism includes a movable rod that advances as much as a predetermined amount when the one operation is input into the operating body, and a restricting portion that restricts the movable rod from retreating, and is configured to regulate the unit closing amount from the opened state toward the closed state when the one operation is input into the operating body.

8. The treatment device according to claim 1, wherein the operating body includes a rotary rod having an external thread, and a projecting body urged to the rotary rod outwardly in a radial direction, and
the interlocking mechanism includes an internal thread into which the external thread is screwed in the handle, and a long hole that is disposed in the handle, through which the projecting body is projectable to the outside and that is long along an axial direction of the rotary rod, and the interlocking mechanism is configured to regulate the unit closing amount from the opened state toward the closed state when the one operation is input into the operating body.

9. The treatment device according to claim 1, wherein the interlocking mechanism includes:
a movable rod that is movable along an axial direction thereof;
a pressing body that advances the movable rod to the handle as much as a predetermined amount when the one operation is input into the operating body; and
a restricting portion that restricts the movable rod from retreating, and
the interlocking mechanism is configured to regulate the unit closing amount for the each one operation.

10. The treatment device according to claim 1, wherein the handle includes a handle main body, and
the interlocking mechanism is configured to be close to the closed state from the opened state of the first and second jaws, every time an operation of bringing the operating body close to the handle main body and separating the operating body from the handle main body is input as one cycle.

11. The treatment device according to claim 10,
wherein the interlocking mechanism is configured to be close to the closed state from the opened state of the first and second jaws, every time an operation of rotating the operating body to the handle once in a predetermined direction is input as one cycle.

12. The treatment device according to claim 1, wherein the interlocking mechanism is configured to reach the closed state from the opened state of the first and second jaws when multiple times of the one operation are repeated.

13. A treatment system comprising:
the treatment device according to claim 1; and
an energy source that allows the energy output portion of the treatment device to output the energy.
